(19) Europäisches Patentamt
European Patent Office
Office européen des brevets

(11) EP 1 091 954 B1

(12) EUROPEAN PATENT SPECIFICATION

(45) Date of publication and mention of the grant of the patent: **12.03.2003 Bulletin 2003/11**

(21) Application number: **99931864.5**

(22) Date of filing: **01.07.1999**

(51) Int Cl.[7]: **C07D 401/04**, C07D 401/14, A61K 31/44, C07B 35/02, C07B 57/00, C07B 55/00

(86) International application number: **PCT/US99/14122**

(87) International publication number: **WO 00/001689 (13.01.2000 Gazette 2000/02)**

(54) **PROCESS FOR PRODUCING (8-CHLORO-3,10-DIBROMO-6,11-DIHYDRO-5H-BENZO 5,6]CYCLOHEPTA[1,2-B]PYRIDIN-11-YL)-1-PIPERIDINE**

VERFAHREN ZUR HERSTELLUNG VON (8-CHLORO-3,10-DIBROMO-6,11-DIHYDRO-5H-BENZO[5,6] CYCLOHEPTA[1,2-B]PYRIDIN-11-YL)-1-PIPERIDIN

PROCEDE DE PRODUCTION DE (8-CHLORO-3,10-DIBROMO-6,11-DIHYDRO-5H-BENZO 5,6]CYCLOHEPTA[1,2-B]PYRIDINE-11-YL)-1-PIPERIDINE

(84) Designated Contracting States:
**AT BE CH CY DE DK ES FI FR GB GR IE IT LI LU MC NL PT SE**
Designated Extension States:
**RO SI**

(30) Priority: **02.07.1998 US 109365**

(43) Date of publication of application: **18.04.2001 Bulletin 2001/16**

(73) Proprietor: **SCHERING CORPORATION**
**Kenilworth, New Jersey 07033-0530 (US)**

(72) Inventors:
- **NJOROGE, F., George**
  **Union, NJ 07083 (US)**
- **VIBULBHAN, Bancha**
  **Kenilworth, NJ 07033 (US)**
- **GIRIJAVALLABHAN, Viyyoor, M.**
  **Parsippany, NJ 07054 (US)**

(74) Representative: **Gross, Ulrich-Maria et al**
**Uexküll & Stolberg,**
**Patentanwälte,**
**Beselerstrasse 4**
**22607 Hamburg (DE)**

(56) References cited:
**WO-A-97/23478** **WO-A-98/58073**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 091 954 B1

## Description

BACKGROUND OF THE INVENTION

Tricyclic compounds useful as inhibitors of farnesyl protein transferase (FPT) are known in the art.

WO97/23478 published July 3, 1997 discloses the preparation of an intermediate useful in the preparation of FPT inhibitors. The intermediate

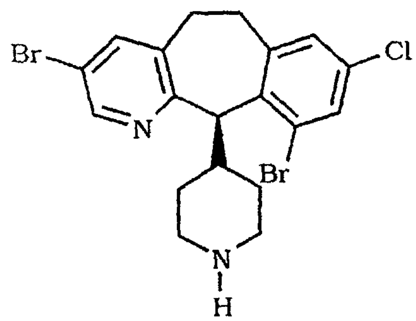

is prepared by reacting

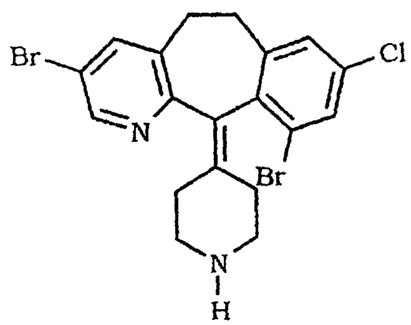

with diisobutylaluminum hydride followed by separation of the racemic mixture using a chiralpak AD column.

Processes which provide improved yields of the above intermediate would be a welcome contribution to the art. This invention provides such a process.

SUMMARY OF THE INVENTION

This invention provides a process for producing a compound of the formula:

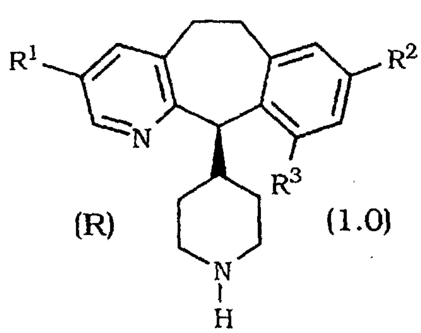

(R) (1.0)

comprising:

(1) separating the atropisomers of

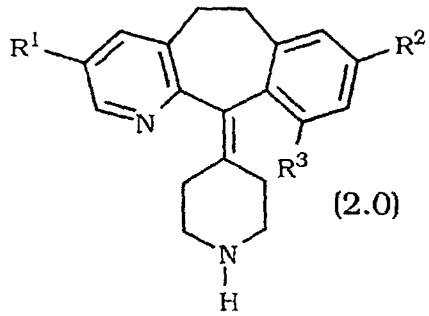

(2.0)

to obtain the atropisomers

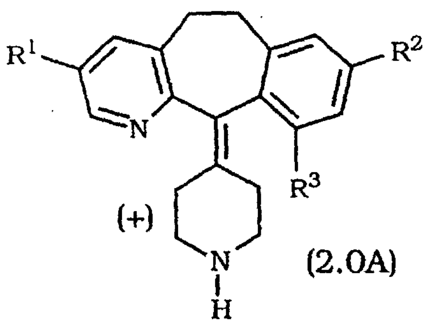

(+) (2.0A)

and

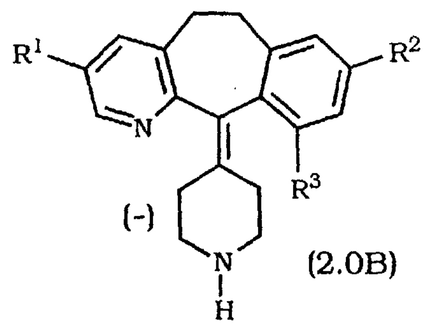

(-) (2.0B)

;

(2) heating the atropisomer of formula 2.0B at a suitable temperature in a suitable solvent to obtain a mixture of atropisomers of formulas 2.0A and 2.0B;
(3) separating the atropisomers of formulas 2.0A and 2.0B of step (2); and
(4) reducing the atropisomer of formula 2.0A to obtain a compound of formula 1.0;

wherein:

$R^1$, $R^2$, and $R^3$ are independently selected from halogen (i.e., Cl, Br, or I), $C_1$ to $C_6$ alkyl or $-OR^4$ wherein $R^4$ is a $C_1$ to $C_6$ alkyl.

Preferably, $R^1$ is Br, $R^2$ is Cl and $R^3$ is Br--i.e., preferably this invention provides a process for producing a compound of the formula:

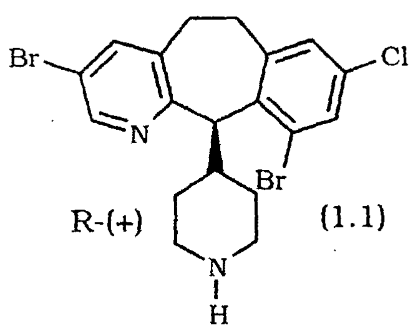

R-(+) (1.1)

comprising:

(1) separating the atropisomers of

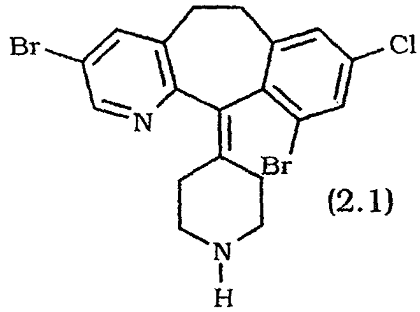

(2.1)

to obtain the atropisomers

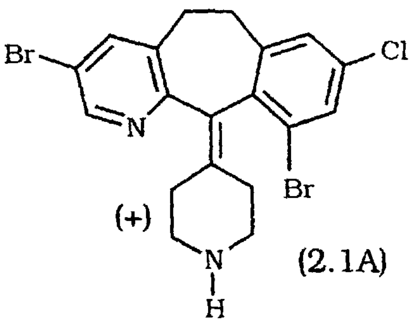

(+) (2.1A)

and

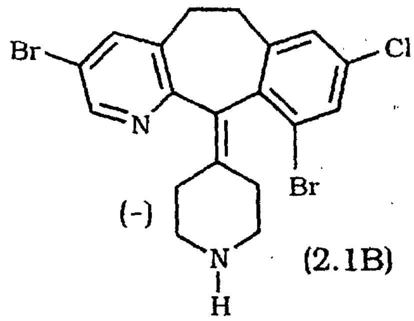

(-) (2.1B)

;

(2) heating the atropisomer of formula 2.1B at a suitable temperature in a suitable solvent to obtain a mixture of atropisomers of formulas 2.1A and 2.1B;

(3) separating the atropisomers of formulas 2.1A and 2.1B of step (2); and

(4) reducing the atropisomer of formula 2.1A to obtain a compound of formula 1.1.

DETAILED DESCRIPTION OF THE INVENTION

The process of this invention provides the compound of formula 1.0 (preferably 1.1) as the specific (R)-isomer--i.e., no racemic mixture (based on C-11) is produced in the reduction step. Those skilled in the art will appreciate that C-11 position in the tricyclic ring is

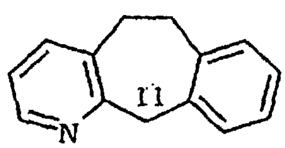

The intermediate compound of formula 1.1 is useful in the preparation of FPT inhibitors disclosed, for example, in WO97/23478. Thus, the compound of formula 1.1 is useful in the preparation of:

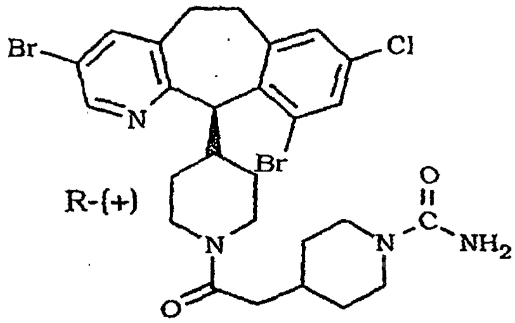

In the process of this invention atropisomer 2.0A obtained in step 1 above can be reduced while additional atropisomer 2.0A is being obtained in steps 2 and 3 above. Thus, atropisomer 2.0A from step 1 above can be reduced as soon as it is obtained. Alternatively, atropisomer 2.0A obtained from step 1 above can be combined with atropisomer 2.0A obtained from steps 2 and 3 above, and the total amount of atropisomer 2.0A can be reduced at one time. Steps 2 and 3 above can be repeated to obtain additional atropisomer 2.0A from atropisomer 2.0B.

Formula 2.0 is separated, in step 1, into its atropisomers using HPLC and a suitable column (i.e., a column that will provide the desired degree of separation in a reasonable amount of time). Preferably, for separating compound 2.1, the column is packed with amylose tris (3,5-dimethylphenyl carbamate) coated on a 10 micron silica gel. This column is commercially available under the tradename Chiralpak AD.

A suitable elution solvent is used to obtain separation of the atropisomers. A suitable solvent is one which provides the desired degree of polarity to sufficiently separate the isomers in a reasonable amount of time. For example, the solvent can comprise: (1) a low boiling alcohol (e.g., isopropanol, methanol, ethanol, mixtures thereof, or the like); (2) a low boiling organic co-solvent (e.g., hexane, pentane, heptane, mixtures thereof, or the like); and (3) an organic base (e.g., diethylamine, diisopropylamine, triethylamine, mixtures thereof, or the like). The solvent, for example, can comprise from 15 to 35% alcohol, and 40 to 85% organic co-solvent, and 0.1 to 1% base, such that the total amout equals 100%v/v. For example, the elution solvent can comprise 15 to 35% isopropyl alcohol, and about 40 to 85% hexane, and 0.1 to 1% diethylamine such that the total amout equals 100%v/v. Preferably, for compound 2.1, the elution solvent comprises 35% isopropyl alcohol and 0.2% diethylamine in hexane.

To convert atropisomer 2.0B to a mixture of atropisomers 2.0A and 2.0B, atropisomer 2.0B is heated at a suitable temperature in a suitable organic solvent. Atropisomer 2.0B is heated to 100 to 200°C in an appropriate high boiling solvent. Generally, atropisomer 2.0B is heated to reflux in the solvent. Solvents are selected from dimethyl formamide, toluene, and and 1,2-dichlorobenzene. Preferably, atropisomer 2.1B is heated in 1,2-dichlorobenzene at a temperature of 150°C.

The atropisomer of formula 2.0A is reduced using a suitable reducing agent. Preferably, diisobutylaluminum hydride is used. The reduction is carried out using conditions well, known to those skilled in the art. For example, atropisomer 2.0A can be dissolved in a suitable organic solvent (e.g., toluene) to which a suitable amount of diisobutylaluminum hydride is added to effectively reduce 2.0A. The solution is then refluxed under nitrogen. The desired product can then be isolated by known separation procedures.

This invention is exemplified by the following example, which should not be construed to limit the scope of the disclosure.

EXAMPLE 1

Step 1

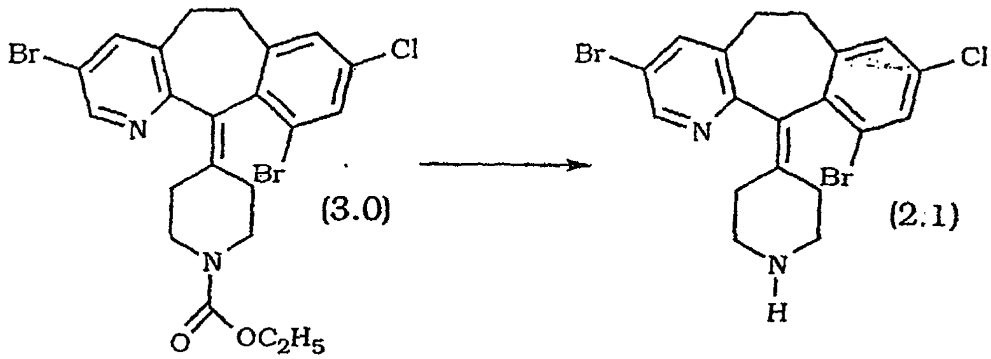

(3.0) (2.1)

To 8 mL of concentrated HCl was added the compound of formula 3.0 (0.7 g, 1.4 mmol). The reaction mixture was refluxed for 16 hours. The reaction mixture was then cooled, poured into an ice bath and basified to pH 10 with aqueous 50% NaOH. The aqueous phase was extracted with $CH_2Cl_2$. Concentration of the organic phase afforded 0.59 g of the compound of formula 2.1 mp=123.9-124.2°C. $^1H$ NMR (200MHz, $CDCl_3$) δ1.99-3.60 (m, 13H), 7.20 (s, 1H), 7.50 (s, 1H), 7.51 (s, 1H), 8.50 (s, 1H). MS m/z (rel intens) 688 (100, $MH^+$).

Step 2

The compound of formula 2.1 obtained in Step 1 was loaded on a Chiracel AD™ column (in HPLC) and eluted with 35% isopropyl alcohol-hexane-containing 0.2% diethylamine to give 4.28 g of atropisomer of formula 2.1A (eluting at retention time 13.04 minutes) and 3.56 g of atropisomer 2.1B (eluting at retention time 51.18 minutes).

Physical chemical data for isomer 2.1A: mp=92-93°C, MS m/z 470 ($MH^+$); $[\alpha]_D^{25}$ = +166.3° (10.02 mg/2mL MeOH).

Physical chemical data for isomer 2.1B: mp=96-97°C, MS m/z 470 ($MH^+$); $[\alpha]_D^{25}$ = -190.2° (9.62 mg/2mL MeOH).

Step 3

To a solution of atropisomer 2.1A (0.38 g, 0.8 mmol) dissolved in toluene (10 mL) was added 0.8 mL (1 eq) of diisobutylaluminum hydride (1 M solution in toluene). the solution was brought to reflux under nitrogen and an additional 1.04 mL (1.3 eq) of 1 M diisobutylaluminum hydride in toluene was added dropwise over 15 minutes. the solution was cooled in an ice-water bath, then mixed with 1 M hydrochloric acid (10 mL). The organic phase was discarded and the aqueous phase was washed with dichloromethane which was also discarded. The aqueous phase was basified with 1 N aqueous sodium hydroxide., extracted with dichloromethane and dried over anhydrous $MgSO_4$. Filtration and concentration in vacuo afforded 0.27 g of compound 1.1 as a white solid. mp=95-96°C. MS (Cl) m/z 469 ($MH^+$). $[\alpha]_D^{25}$ = +51.9° (7.71 mg/2mL MeOH). $^1H$ NMR (200MHz, $CDCl_3$) δ (ppm) 1.16-1.83 (m, 5H), 2.16-2.57 (m, 3H), 2.69-3.17 (m, 1H), 3.65 (m, 1H), 4.91 (d, 1H, J=10Hz), 7.13 (d, 1H, J=2Hz), 7.50 (d, 1H, J=2Hz), 7.54 (d, 1H, J=2Hz), 8.45 (d, 1H, J=2Hz).

Step 4

Atropisomer 2.1B (0.25g) was converted to atropisomer 2.1A by heating in 4 mL of 1,2-dichlorobenzene at 150°C. After 7 days 45% of atropisomer 2.1B was converted to atropisomer 2.1A.

While the present invention has been described in conjunction with the specific embodiments set forth above, many alternatives, modifications and variations thereof will be apparent to those of ordinary skill in the art.

## Claims

1. A process for producing a compound of the formula:

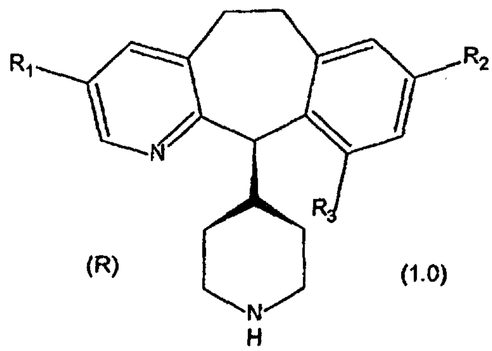

(R) (1.0)

comprising:

I.

(1) separating the atropisomers of

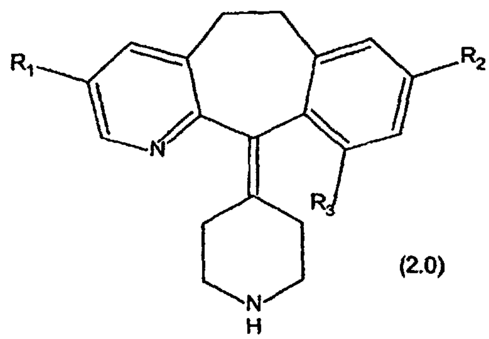

(2.0)

by HPLC to obtain the atropisomers

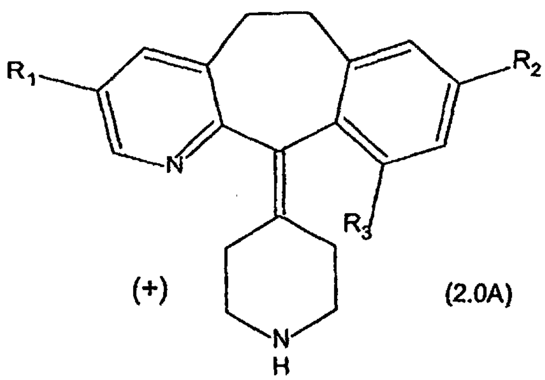

(+) (2.0A)

and

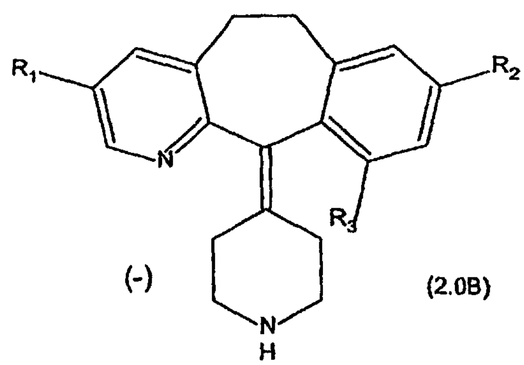

(-) (2.0B)

(2) heating the atropisomer of formula 2.0B at a temperature of 100 to 200°C in a solvent selected from dimethyl formamide, toluene or 1,2-dichlorobenzene to obtain a mixture of atropisomers of formulas 2.0A and 2.0B;
(3) separating the atropisomers of formulas 2.0A and 2.0B of step (2) by HPLC; and
(4) reducing the atropisomer of formula 2.0A to obtain a compound of formula 1.0; or

II.

(1) separating the atropisomers of

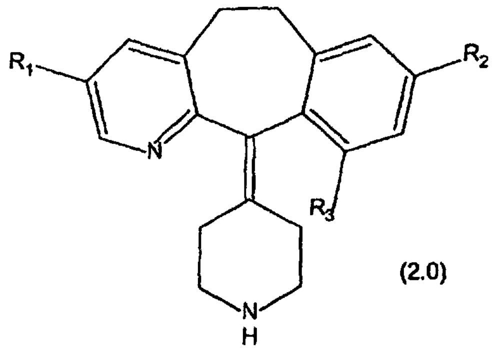

(2.0)

by HPLC to obtain the atropisomers

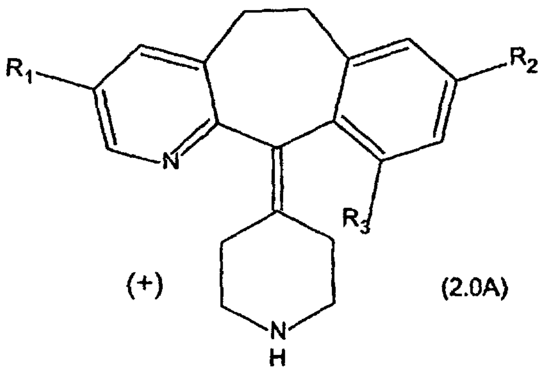

(+) (2.0A)

and

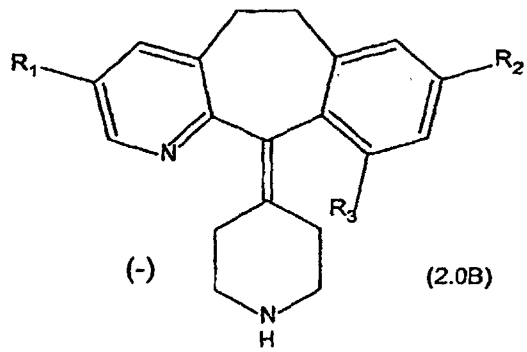

(-) (2.0B)

(2) reducing the atropisomer of formula 2.0A to obtain a compound of formula 1.0 ;

(3) heating the atropisomer of formula 2.0B at a temperature of 100 to 200°C in a solvent selected from dimethyl formamide, toluene or 1,2-dichlorobenzene to obtain a mixture of atropisomers of formulas 2.0A and 2.0B ;

(4) separating the atropisomers of formulas 2.0A and 2.0B of step (3) by HPLC; and

(5) reducing the atropisomer of formula 2.0A obtained in Step (4) to obtain a compound of formula 1.0;

wherein:

R1, R2, and R3 are independently selected from halogen, $C_1$ to $C_6$ alkyl or -OR4 wherein R4 is a $C_1$ to $C_6$ alkyl.

**2.** A process for producing a compound of the formula:

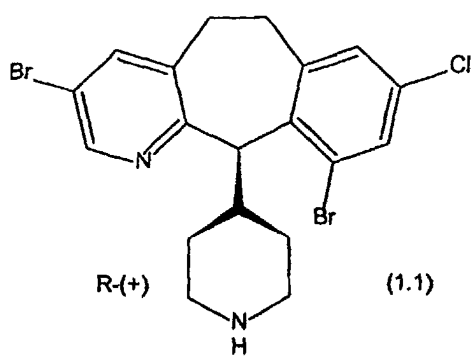

R-(+) (1.1)

comprising:

(1) separating the atropisomers of

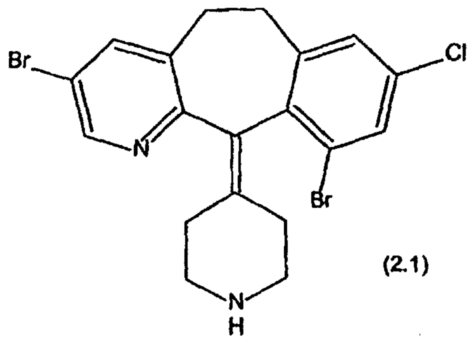

(2.1)

by HPLC to obtain the atropisomers

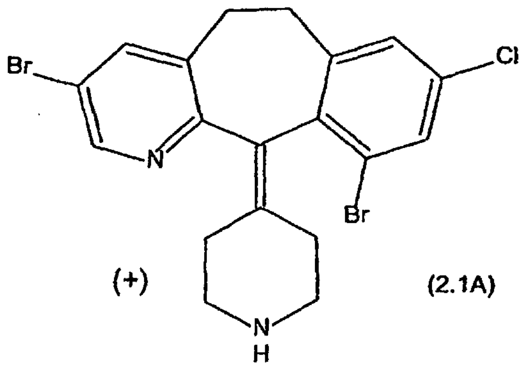

(+) (2.1A)

and

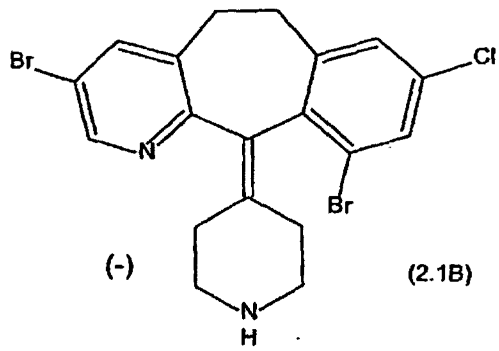

(-) (2.1B)

(2) heating the atropisomer of formula 2.1B at a temperature of 100 to 200°C in a solvent selected from dimethyl formamide, toluene or 1,2-dichlorobenzene to obtain a mixture of atropisomers of formulas 2.1A and 2.1B ;
(3) separating the atropisomers of formulas 2.1A and 2.1B of step (2) by HPLC; and
(4) reducing the atropisomer of formula 2.1A to obtain a compound of formula 1.1

wherein the HPLC column used in steps (1) and (3) comprises amylose tris (3,5-dimethylphenyl carbamate) coated on a 10 micron silica gel substrate and wherein an elution solvent is used comprising 15 to 35% isopropyl alcohol, and 40 to 85% hexane, and 0.1 to 1% diethylamine such that the total amout equals 100%v/v.

**3.** The process of Claim 2 wherein elution solvent comprises 35%v/v isopropyl alcohol and 0.2%v/v diethylamine in hexane.

**4.** The process of Claim 2 wherein atropisomer 2.1B is heated in 1,2-dichlorobenzene to obtain the mixture of atropisomers 2.1A and 2.1B.

**5.** The process of Claim 4 wherein atropisomer 2.1B is heated at 150° C.

**6.** The process of Claim 2 wherein atropisomer 2.1A is reduced using diisobutylaluminum hydride.

**7.** The process of Claim 2 wherein: (a) atropisomer 2.1B is heated at 150°C in 1,2-dichlorobenzene to obtain the mixture of atropisomers 2.1A and 2.1B ; and (b) atropisomer 2.1 A is reduced using diisobutylaluminum hydride.

**8.** The process of Claim 7 wherein elution solvent comprises 35%v/v isopropyl alcohol and 0.2%v/v diethylamine in hexane.

**9.** A process for producing a compound of the formula:

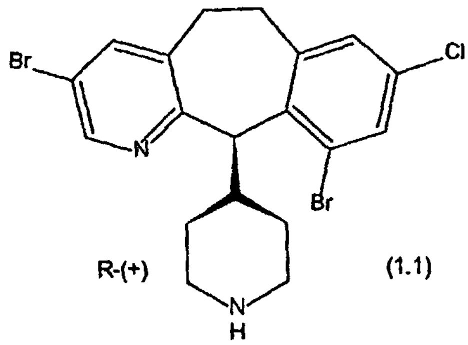

R-(+) (1.1)

comprising:

(1) separating the atropisomers of

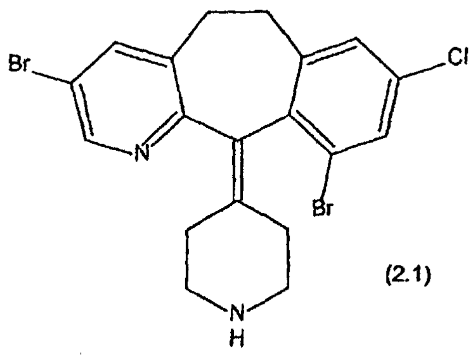

(2.1)

by HPLC to obtain the atropisomers

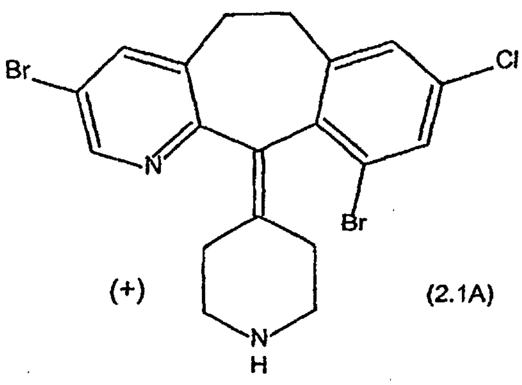

(+) (2.1A)

and

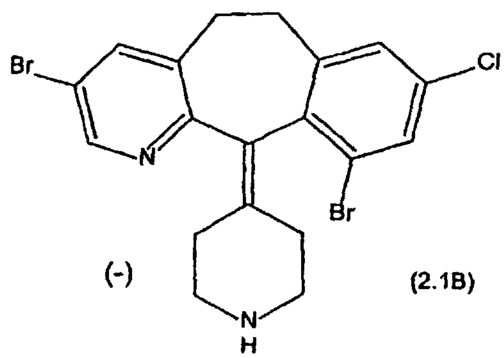

(-) (2.1B)

(2) reducing the atropisomer of formula 2.1A to obtain a compound of formula 1.1;
(3) heating the atropisomer of formula 2.1B at a temperature of 100 to 200°C in a solvent selected from dimethyl formamide, toluene or 1,2-dichlorobenzene to obtain a mixture of atropisomers of formulas 2.1A and 2.1B;
(4) separating the atropisomers of formulas 2.1A and 2.1B of step (3) by HPLC; and
(5) reducing the atropisomer of formula 2.1A obtained in Step (4) to obtain a compound of formula 1. 1;

wherein the HPLC column used in steps (1) and (4) comprises amylose tris (3,5-dimethylphenyl carbamate) coated on a 10 micron silica gel substrate and wherein an elution solvent is used comprising 15 to 35% isopropyl alcohol, and 40 to 85% hexane, and 0.1 to 1% diethylamine such that the total amout equals 100%v/v.

**10.** The process of Claim 9 wherein said elution solvent comprises 35%v/v isopropyl alcohol and 0.2%v/v diethylamine in hexane.

11. The process of Claim 9 wherein atropisomer 2.1B is heated in 1,2-dichlorobenzene to obtain the mixture of atropisomers 2.1A and 2.1B.

12. The process of Claim 11 wherein atropisomer 2.1B is heated at 150° C.

13. The process of Claim 9 wherein atropisomer 2.1A is reduced using diisobutylaluminum hydzide.

14. The process of Claim 9 wherein: (a) atropisomer 2.1B is heated at 150° C in 1,2-dichlorobenzene to obtain the mixture of atropisomers 2.1A and 2.1B ; and (b) atropisomer 2.1A is reduced using diisobutylaluminum hydride.

15. The process of Claim 14 wherein elution solvent comprises 35%v/v isopropyl alcohol and 0.2%v/v diethylamine in hexane.

**Patentansprüche**

1. Verfahren zur Herstellung einer Verbindung mit der Formel

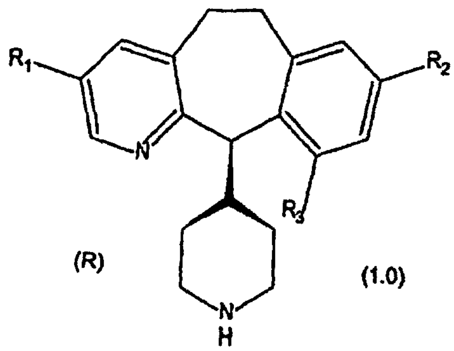

(R) (1.0)

, bei dem

I.

(1) die Atropisomere von

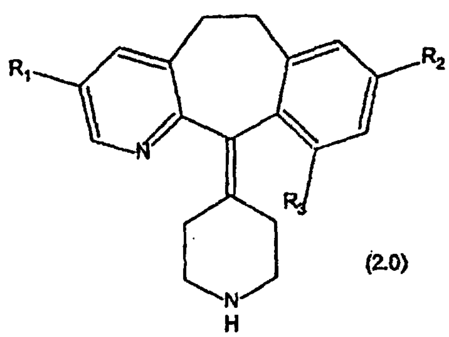

(2.0)

mittels HPLC getrennt werden, um die Atropisomere

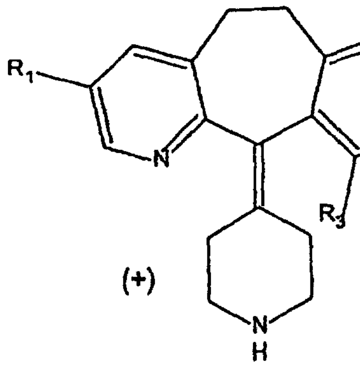

(+) (2.0A) und

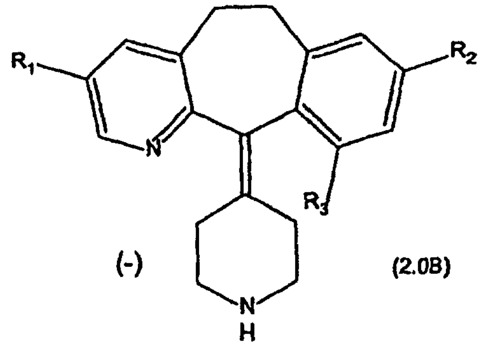

(-) (2.0B)

zu erhalten;

(2) das Atropisomere mit der Formel 2.0B in einem Lösungsmittel ausgewählt aus Dimethylformamid, Toluol oder 1,2-Dichlorbenzol auf eine Temperatur von 100 bis 200°C erwärmt wird, um eine Mischung der Atropisomere mit den Formeln 2.0A und 2.0B zu erhalten;

(3) die Atropisomere mit den Formeln 2.0A und 2.0B der Stufe (2) mittels HPLC getrennt werden; und

(4) das Atropisomer mit der Formel 2.0A reduziert wird, um eine Verbindung mit der Formel 1.0 zu erhalten; oder

II.

(1) die Atropisomere von

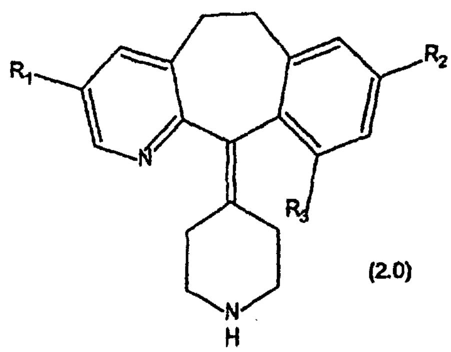

(2.0)

mittels HPLC getrennt werden, um die Atropisomere

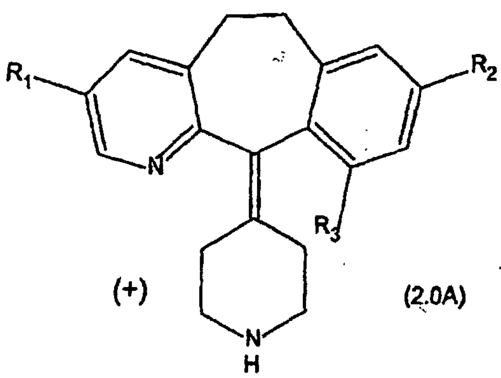

(+) (2.0A) und

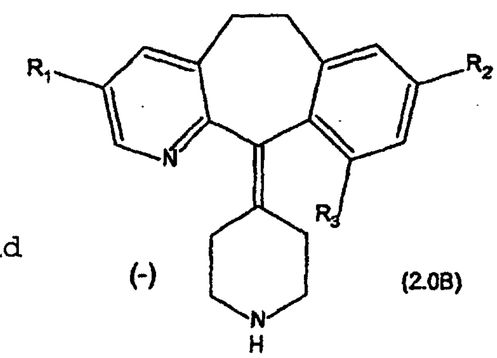

(-) (2.0B)

zu erhalten;

(2) das Atropisomer mit der Formel 2.0 A reduziert wird, um eine Verbindung mit der Formel 1.0 zu erhalten;

(3) das Atropisomer mit der Formel 2.0B in einem Lösungsmittel ausgewählt aus Dimethylformamid, Toluol

oder 1,2-Dichlorbenzol auf eine Temperatur von 100 bis 200°C erwärmt wird, um eine Mischung der Atropisomere der Formeln 2.0A und 2.0B zu erhalten;
(4) die Atropisomere mit den Formeln 2.0A und 2.0B von Stufe (3) mittels HPLC getrennt werden; und
(5) das Atropisomer mit der Formel 2.0A, das in Stufe (4) erhalten wurde, reduziert wird, um eine Verbindung mit der Formel 1.0 zu erhalten;

wobei
R1, R2 und R3 unabhängig ausgewählt sind aus Halogen, $C_1$- bis $C_6$-Alkyl oder -OR4, wobei R4 $C_1$- bis $C_6$-Alkyl ist.

**2.** Verfahren zur Herstellung einer Verbindung mit der Formel

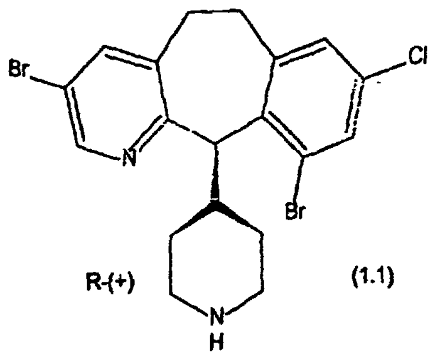

R-(+) (1.1)

, bei dem

(1) die Atropisomere von

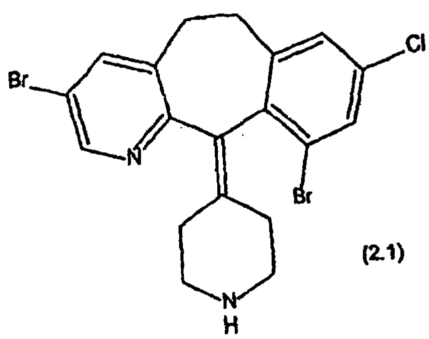

(2.1)

mittels HPLC getrennt werden, um die Atropisomere

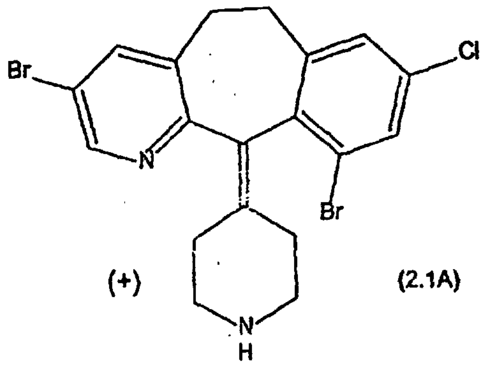
(+) (2.1A) und

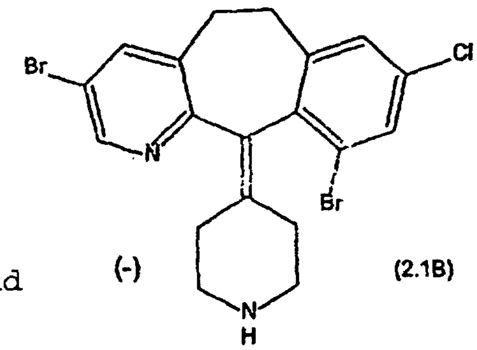
(-) (2.1B)

zu erhalten;

(2) das Atropisomere mit der Formel 2.1B in einem Lösungsmittel ausgewählt aus Dimethylformamid, Toluol oder 1,2-Dichlorbenzol erwärmt wird, um eine Mischung der Atropisomere mit den Formel 2.1A und 2.1B zu erhalten;

(3) die Atropisomere mit den Formeln 2.1A und 2.1B von Stufe (2) mittels HPLC getrennt werden; und

(4) das Atropisomer mit der Formel 2.1A reduziert wird, um eine Verbindung mit der Formel 1.1 zu erhalten,

wobei die in den Stufen (1) und (3) verwendete HPLC-Säule Amylosetris(3,5-dimethylphenylcarbamat) als Beschichtung auf einem 10 µm Silikagelsubstrat enthält und ein Eluierungslösungsmittel verwendet wird, das 15 bis 35 % Isopropylalkohol und 40 bis 85 % Hexan und 0,1 bis 1 % Diethylamin enthält, so dass die Gesamtmenge gleich 100 % Vol./Vol. beträgt.

3. Verfahren nach Anspruch 2, bei dem das Eluierungslösungsmittel 35 % Vol./Vol. Isopropylalkohol und 0,2 % Vol. %Vol. Diethylamin in Hexan enthält.

4. Verfahren nach Anspruch 2, bei dem Atropisomer 2.1B in 1,2-Dichlorbenzol erwärmt wird, um die Mischung der Atropisomere 2.1A und 2.1B zu erhalten.

5. Verfahren nach Anspruch 4, bei dem Atropisomer 2.1B auf 150°C erwärmt wird.

6. Verfahren nach Anspruch 2, bei dem Atropisomer 2.1A unter Verwendung von Diisobutylaluminiumhydrid reduziert wird

7. Verfahren nach Anspruch 2, bei dem (a) Atropisomer 2.1B in 1,2-Dichlorbenzol auf 150°C erwärmt wird, um die Mischung der Atropisomere 2.1A und 2.1B zu erhalten und (b) Atropisomer 2.1A unter Verwendung von Diisobutylaluminiumhydrid reduziert wird.

8. Verfahren nach Anspruch 7, bei dem das Eluierungslösungsmittel 35 % Vol./Vol. Isopropylalkohol und 0,2 % Vol./Vol. Diethylamin in Hexan enthält.

9. Verfahren zur Herstellung einer Verbindung mit der Formel:

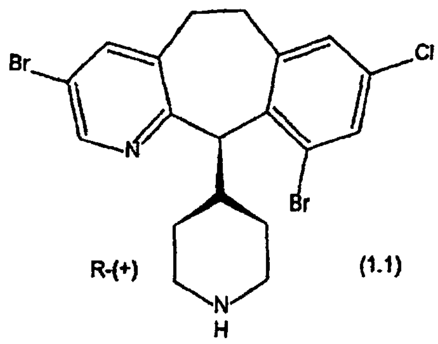

R-(+) (1.1)

, bei dem

(1) die Atropisomere von

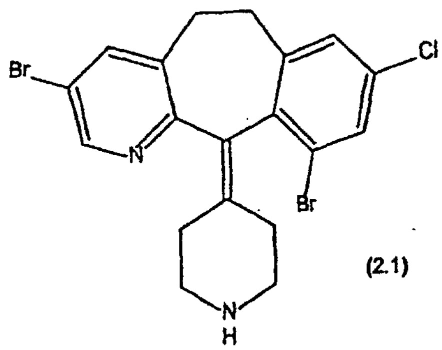

(2.1)

mittels HPLC getrennt werden, um die Atropisomere

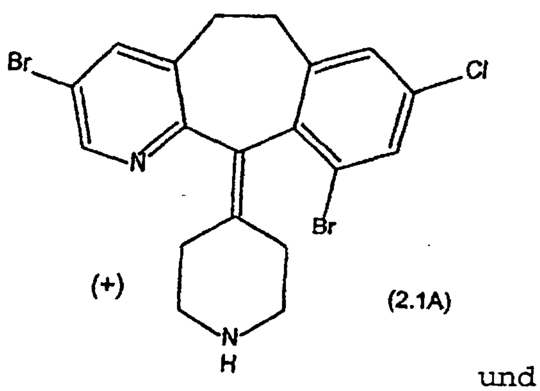

(+) (2.1A)

und

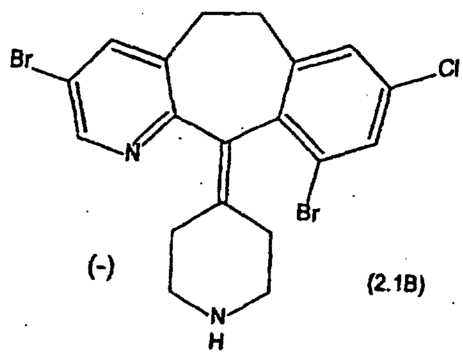

(-) (2.1B)

zu erhalten;

(2) das Atropisomer mit der Formel 2.1A reduziert wird, um eine Verbindung mit der Formel 1.1 zu erhalten;

(3) das Atropisomer mit der Formel 2.1B in einem Lösungsmittel ausgewählt aus Dimethylformamid, Toluol oder 1,2-Dichlorbenzol auf eine Temperatur von 100 bis 200°C erwärmt wird, um eine Mischung der Atropisomere mit den Formeln 2.1A und 2.1B zu erhalten;

(4) die Atropisomere mit den Formeln 2.1A und 2.1B aus Stufe (3) mittels HPLC getrennt werden; und

(5) das Atropisomer mit der Formel 2.1A, das in Stufe (4) erhalten wurde, reduziert wird, um eine Verbindung mit der Formel 1.1 zu erhalten;

wobei die in den Stufen (1) und (4) verwendete HPLC-Säule Amylosetris(3,5-dimethylphenylcarbamat) als Beschichtung auf einem 10 µm Silikagelsubstrat enthält und ein Eluierungslösungsmittel verwendet wird, das 15 bis

35 % Isopropylalkohol und 40 bis 85 % Hexan und 0,1 bis 1 % Diethylamin enthält, so dass die Gesamtmenge gleich 100 % Vol./Vol. beträgt.

**10.** Verfahren nach Anspruch 9, bei dem das Eluierungslösungsmittel 35 % Vol./Vol. Isopropylalkohol und 0,2 % Vol./ Vol. Diethylamin in Hexan enthält.

**11.** Verfahren nach Anspruch 9, bei dem Atropisomer 2.1B in 1,2-Dichlorbenzöl erwärmt wird, um die Mischung von Atropisomeren 2.1A und 2.1B zu erhalten.

**12.** Verfahren nach Anspruch 11, bei dem Atropisomer 2.1B auf 150°C erwärmt wird.

**13.** Verfahren nach Anspruch 9, bei dem Atropisomer 2.1A unter Verwendung von Diisobutylaluminiumhydrid reduziert wird.

**14.** Verfahren nach Anspruch 9, bei dem (a) Atropisomer 2.1B in 1,2-Dichlorbenzol auf 150°C erwärmt wird, um die Mischung der Atropisomere 2.1A und 2.1B zu erhalten, und (b) Atropisomer 2.1A unter Verwendung von Diisobutylaluminiumhydrid reduziert wird.

**15.** Verfahren nach Anspruch 14, bei dem das Eluierungslösungsmittel 35 % Vol./Vol. Isopropylalkohol und 0,2 % Vol./ Vol. Diethylamin in Hexan enthält.

**Revendications**

**1.** Procédé de préparation d'un composé de formule :

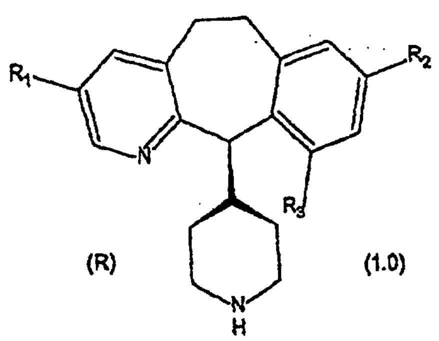

(R) (1.0)

lequel procédé comporte :

A)

1) le fait de séparer les atropisomères de

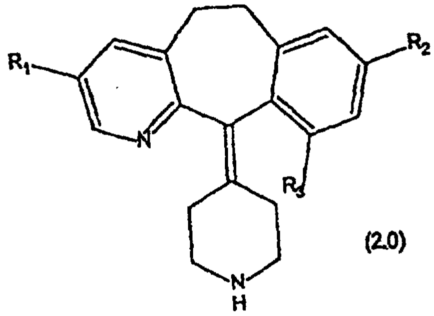

(2.0)

par HPLC, pour obtenir les atropisomères

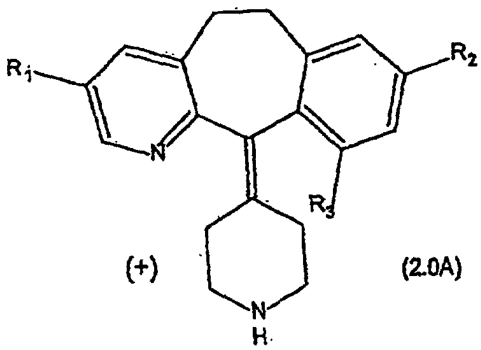

(+) (2.0A) et

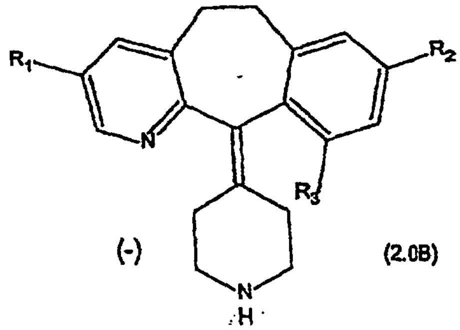

(-) (2.0B)

2) le fait de chauffer l'atropisomère de formule 2.0B à une température de 100 à 200 °C, dans un solvant choisi parmi du diméthylformamide, du toluène et du 1,2-dichlorobenzène, pour obtenir un mélange des atropisomères de formules 2.0A et 2.0B ;
3) le fait de séparer par HPLC les atropisomères de formules 2.0A et 2.0B obtenus dans l'étape (2) ;
4) et le fait de réduire l'atropisomère de formule 2.0A pour obtenir un composé de formule 1.0 ; ou bien

II)

1) le fait de séparer les atropisomères de

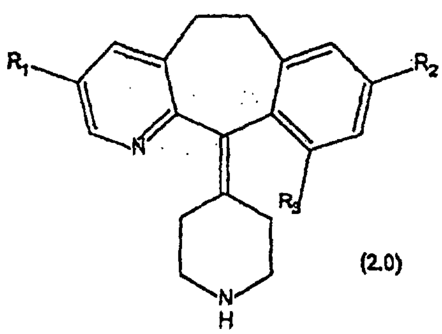

(2.0)

par HPLC, pour obtenir les atropisomères

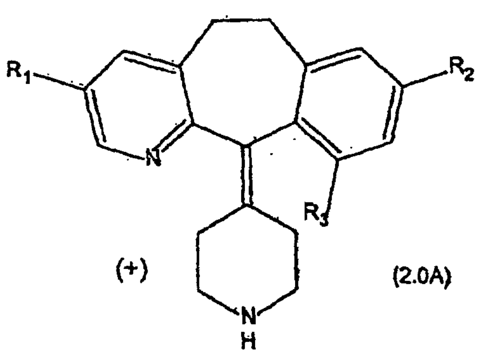

(+) (2.0A) et

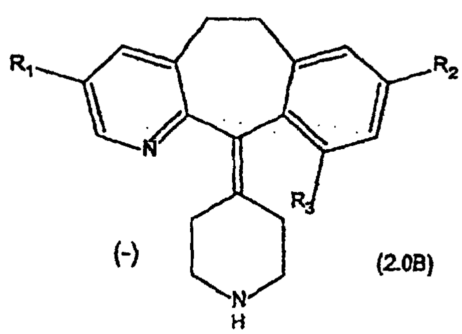

(-) (2.0B)

2) le fait de réduire l'atropisomère de formule 2.0A pour obtenir un composé de formule 1.0 ;
3) le fait de chauffer l'atropisomère de formule 2.0B à une température de 100 à 200 °C, dans un solvant choisi parmi du diméthylformamide, du toluène et du 1,2-dichlorobenzène, pour obtenir un mélange des atropisomères de formules 2.0A et 2.0B ;
4) le fait de séparer par HPLC les atropisomères de formules 2.0A et 2.0B obtenus dans l'étape (3) ;

5) et le fait de réduire l'atropisomère de formule 2.0A pour obtenir un composé de formule 1.0 ;

$R_1$, $R_2$ et $R_3$ représentant chacun, indépendamment, un atome d'halogène, un groupe alkyle en $C_{1-6}$ ou un groupe $-OR_4$ où $R_4$ représente un groupe alkyle en $C_{1-6}$.

**2.** Procédé de préparation d'un composé de formule :

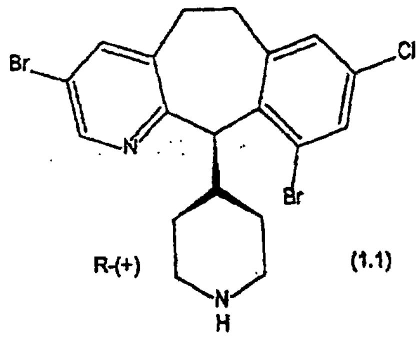

R-(+) (1.1)

lequel procédé comporte :

A)

1) le fait de séparer les atropisomères de

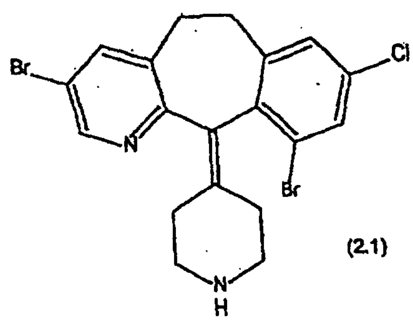

(2.1)

par HPLC, pour obtenir les atropisomères

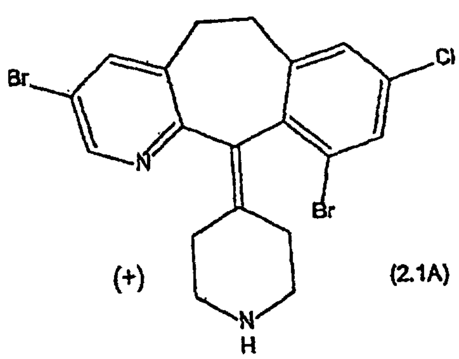

(+) (2.1A) et

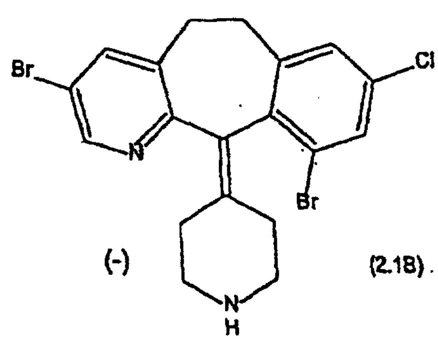

(-) (2.1B).

2) le fait de chauffer l'atropisomère de formule 2.1B à une température de 100 à 200 °C, dans un solvant choisi parmi du diméthylformamide, du toluène et du 1,2-dichlorobenzène, pour obtenir un mélange des atropisomères de formules 2.1A et 2.1B ;

3) le fait de séparer par HPLC les atropisomères de formules 2.1A et 2.1B obtenus dans l'étape (2) ;

4) et le fait de réduire l'atropisomère de formule 2.1A pour obtenir un composé de formule 1.1 ;

et dans lequel procédé la colonne d'HPLC employée dans les étapes (1) et (3) comprend du tris(3,5-diméthylphényl-carbamate) d'amylose enrobant un substrat de gel de silice (10 μm) et le solvant servant à l'élution est constitué de 15 à 35 % d'isopropanol, de 40 à 85 % d'hexane et de 0,1 à 1 % de diéthylamine, pour un total de 100 % v/v.

**3.** Procédé conforme à la revendication 2, dans lequel le solvant d'élution est constitué de 35 % v/v d'isopropanol et de 0,2 % v/v de diéthylamine dans de l'hexane.

**4.** Procédé conforme à la revendication 2, dans lequel on chauffe l'atropisomère 2.1B dans du 1,2-dichlorobenzène pour obtenir un mélange des atropisomères 2.1A et 2.1B.

**5.** Procédé conforme à la revendication 4, dans lequel on chauffe l'atropisomère 2.1B à 150 °C.

**6.** Procédé conforme à la revendication 2, dans lequel on réduit l'atropisomère 2.1A avec de l'hydrure de diisobutyl-aluminium.

**7.** Procédé conforme à la revendication 2, dans lequel :

a) on chauffe l'atropisomère 2.1B à 150 °C dans du 1,2-dichlorobenzène pour obtenir un mélange des atropisomères 2.1A et 2.1B ;
b) et l'on réduit l'atropisomère 2.1A avec de l'hydrure de diisobutyl-aluminium.

**8.** Procédé conforme à la revendication 7, dans lequel le solvant d'élution est constitué de 35 % v/v d'isopropanol et de 0,2 % v/v de diéthylamine dans de l'hexane.

**9.** Procédé de préparation d'un composé de formule :

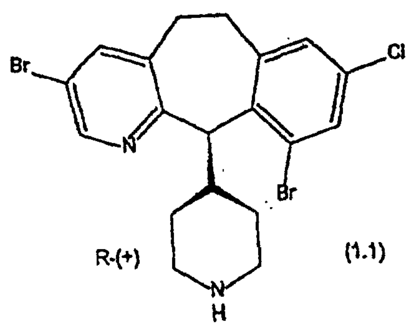

R-(+) (1.1)

lequel procédé comporte :

1) le fait de séparer les atropisomères de

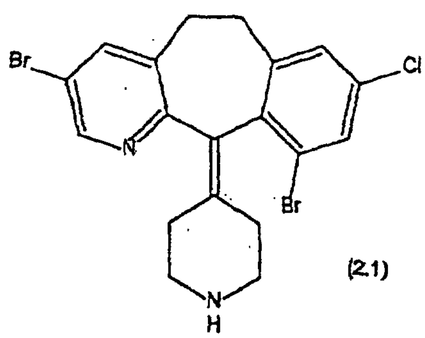

(2.1)

par HPLC, pour obtenir les atropisomères

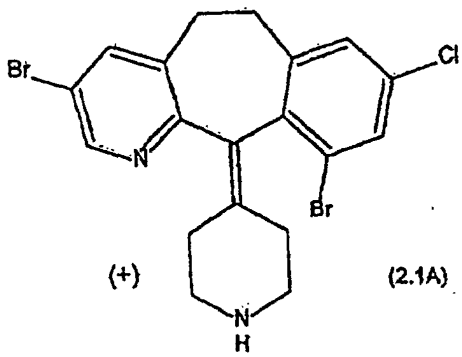

(+) (2.1A) et

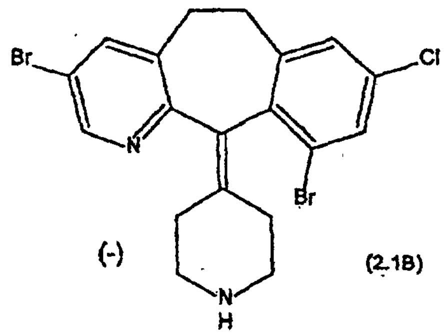

(-) (2.1B)

2) le fait de réduire l'atropisomère de formule 2.1A pour obtenir un composé de formule 1.1;
3) le fait de chauffer l'atropisomère de formule 2.1B à une température de 100 à 200 °C, dans un solvant choisi parmi du diméthylformamide, du toluène et du 1,2-dichlorobenzène, pour obtenir un mélange des atropisomères de formules 2.1A et 2.1B ;
4) le fait de séparer par HPLC les atropisomères de formules 2.1A et 2.1B obtenus dans l'étape (3) ;
5) et le fait de réduire l'atropisomère de formule 2.1A pour obtenir un composé de formule 1.1 ;

et dans lequel procédé la colonne d'HPLC employée dans les étapes (1) et (4) comprend du tris(3,5-diméthylphényl-carbamate) d'amylose enrobant un substrat de gel de silice (10 µm) et le solvant servant à l'élution est constitué de 15 à 35 % d'isopropanol, de 40 à 85 % d'hexane et de 0,1 à 1 % de diéthylamine, pour un total de 100 % v/v.

**10.** Procédé conforme à la revendication 9, dans lequel le solvant d'élution est constitué de 35 % v/v d'isopropanol et de 0,2 % v/v de diéthylamine dans de l'hexane.

**11.** Procédé conforme à la revendication 9, dans lequel on chauffe l'atropisomère 2.1B dans du 1,2-dichlorobenzène pour obtenir un mélange des atropisomères 2.1A et 2.1B.

**12.** Procédé conforme à la revendication 11, dans lequel on chauffe l'atropisomère 2.1B à 150 °C.

**13.** Procédé conforme à la revendication 9, dans lequel on réduit l'atropisomère 2.1A avec de l'hydrure de diisobutylaluminium.

**14.** Procédé conforme à la revendication 9, dans lequel :

a) on chauffe l'atropisomère 2.1B à 150 °C dans du 1,2-dichlorobenzène pour obtenir un mélange des atropisomères 2.1A et 2.1B ;
b) et l'on réduit l'atropisomère 2.1A avec de l'hydrure de diisobutyl-aluminium.

**15.** Procédé conforme à la revendication 14, dans lequel le solvant d'élution est constitué de 35 % v/v d'isopropanol et de 0,2 % v/v de diéthylamine dans de l'hexane.